(19) Europäisches Patentamt
European Patent Office
Office européen des brevets

(11) **EP 4 485 298 A1**

(12) **EUROPEAN PATENT APPLICATION**
published in accordance with Art. 153(4) EPC

(43) Date of publication:
**01.01.2025 Bulletin 2025/01**

(21) Application number: **23759960.0**

(22) Date of filing: **21.02.2023**

(51) International Patent Classification (IPC):
**G06N 99/00** (2019.01)

(52) Cooperative Patent Classification (CPC):
**G06N 99/00**

(86) International application number:
**PCT/JP2023/006148**

(87) International publication number:
**WO 2023/162959 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.02.2022 JP 2022025318**

(71) Applicant: **Nitto Denko Corporation**
**Ibaraki-shi, Osaka 567-8680 (JP)**

(72) Inventors:
• **YOSHIDA, Kei**
**Ibaraki-shi, Osaka 567-8680 (JP)**

• **KIGAWA, Yoichi**
**Ibaraki-shi, Osaka 567-8680 (JP)**
• **MATSUOKA, Satoshi**
**Ibaraki-shi, Osaka 567-8680 (JP)**
• **MATSUNAMI, Jun**
**Ibaraki-shi, Osaka 567-8680 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **CONDITION OPTIMIZATION DEVICE, CONDITION OPTIMIZATION METHOD, AND PROGRAM**

(57)    A condition value is efficiently optimized. A condition optimization device includes: an actual reaction value obtainment unit configured to obtain an actual reaction value by observing a predetermined physical property value at a predetermined condition value; a condition value calculation unit configured to calculate a new condition value by Bayesian optimization; an optimum condition value prediction unit configured to predict, by a response surface method, a best physical property value and an optimum condition value at which the best physical property value is obtainable; a first determination unit configured to determine whether or not convergence has occurred based on the condition value corresponding to the actual reaction value and based on the new condition value; and a second determination unit configured to determine, upon the first determination unit determining that the convergence has occurred, whether or not convergence has occurred based on a difference between the physical property value observed at the optimum condition value and the best physical property value.

FIG.3

**Description**

TECHNICAL FIELD

The present invention relates to condition optimization devices, condition optimization methods, and programs.

BACKGROUND ART

Bayesian optimization is used for optimizing condition values. According to Bayesian optimization, condition values to be experimented next can be obtained based on a plurality of actual reaction values obtained in accordance with different condition values.

Meanwhile, in order to find optimum condition values with a small number of experiments, the response surface method by a support vector machine is used. According to the response surface method, the best actual reaction values at certain condition values can be predicted.

For example, Patent Document 1 discloses a technique of evaluating a combination of a plurality of parameters by Bayesian optimization, thereby efficiently designing a device.

RELATED ART DOCUMENTS

PATENT DOCUMENTS

Patent Document 1: Japanese Unexamined Patent Application Publication No. 2019-215750

SUMMARY OF THE INVENTION

PROBLEMS TO BE SOLVED BY THE INVENTION

However, in Bayesian optimization, it is necessary to perform many tests in order to determine whether or not the condition values have been optimized. Also, in the response surface method, the experimenter needs to set in advance the condition values to be experimented next.

In view of the above technical issues, it is an object of one aspect of the present invention to efficiently optimize condition values.

MEANS FOR SOLVING THE PROBLEMS

In order to address the above issues, a condition optimization device according to one aspect of the present invention includes: an actual reaction value obtainment unit configured to obtain an actual reaction value by observing a predetermined physical property value at a predetermined condition value; a condition value calculation unit configured to calculate a new condition value by Bayesian optimization; an optimum condition value prediction unit configured to predict, by a response surface method, a best physical property value and an optimum condition value at which the best physical property value is obtainable; a first determination unit configured to determine whether or not convergence has occurred based on the condition value corresponding to the actual reaction value and based on the new condition value; and a second determination unit configured to determine, upon the first determination unit determining that the convergence has occurred, whether or not convergence has occurred based on a difference between the physical property value observed at the optimum condition value and the best physical property value.

ADVANTAGEOUS EFFECTS OF THE INVENTION

According to one aspect of the present invention, it is possible to efficiently optimize condition values.

BRIEF DESCRIPTION OF THE DRAWINGS

[FIG. 1] FIG. 1 is a diagram illustrating an example of an overall configuration of a condition optimization system according to an embodiment.
[FIG. 2] FIG. 2 is a diagram illustrating an example of a hardware configuration of a computer according to an embodiment.

[FIG. 3] FIG. 3 is a diagram illustrating an example of a functional configuration of the condition optimization system according to the embodiment.

[FIG. 4] FIG. 4 is a diagram illustrating an example of a process of a condition optimization method according to a first embodiment.

[FIG. 5] FIG. 5 is a diagram illustrating an example of condition values in accordance with design of experiments.

[FIG. 6] FIG. 6 is a diagram illustrating an example of a process of a condition optimization method according to a second embodiment.

[FIG. 7] FIG. 7 is a table illustrating an example of actual reaction values according to an embodiment.

DETAILED DESCRIPTION OF THE INVENTION

Hereinafter, embodiments of the present invention will be described with reference to the attached drawings. In the present specification and drawings, components having substantially the same functional configuration are denoted by the same reference numerals, and thus duplicate description thereof will be omitted.

[First embodiment]

The first embodiment of the present invention is a condition optimization system configured to optimize a condition value based on accumulated actual reaction values. The condition optimization system according to the present embodiment calculates a condition value to be experimented next by Bayesian optimization, and determines whether or not the condition value has converged utilizing the response surface method by a support vector machine (hereinafter referred to simply as "response surface method"). Thus, according to the condition optimization system according to the present embodiment, the condition value can be efficiently optimized with a small number of experiments.

<Overall configuration of condition optimization system>

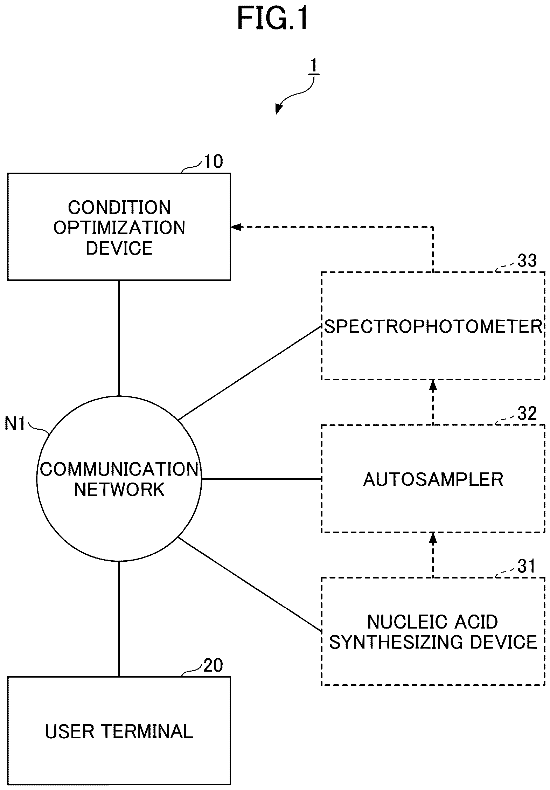

First, the overall configuration of the condition optimization system according to the present embodiment will be described with reference to FIG. 1. FIG. 1 is a block diagram illustrating an example of the overall configuration of the condition optimization system according to the present embodiment.

As illustrated in FIG. 1, a condition optimization system 1 according to the present embodiment includes a condition optimization device 10 and a user terminal 20. The condition optimization system 1 according to the present embodiment may include a nucleic acid synthesizing device 31, an autosampler 32, and a spectrophotometer 33. The condition optimization device 10, the user terminal 20, the nucleic acid synthesizing device 31, the autosampler 32, and the spectrophotometer 33 are connected to enable data communication via a communication network N1, such as a Local Area Network (LAN), the Internet, or the like.

The condition optimization device 10 is an information processing device, such as a Personal Computer (PC), a workstation, a server, or the like, configured to optimize the condition value based on the accumulated actual reaction values. The condition optimization device 10 accumulates the actual reaction values input from the user terminal 20, and outputs a condition value to be experimented next to the user terminal 20 based on the accumulated actual reaction values. The condition optimization device 10 determines whether or not the condition value has converged every time the condition value is calculated, and outputs an optimized condition value to the user terminal 20 upon determining that the condition value has converged.

The user terminal 20 is an information processing device used by a user, such as a Personal Computer (PC), a workstation, a server, or the like. The user terminal 20 shows the user the condition value output by the condition optimization device 10, and inputs an actual reaction value observed in accordance with the condition value to the condition optimization device 10.

The nucleic acid synthesizing device 31, the autosampler 32, and the spectrophotometer 33 are a configuration that automatically obtains the actual reaction value when optimizing the condition values of nucleic acid synthesis. The nucleic acid synthesizing device 31 is, for example, a device configured to synthesize DNA oligonucleotides. The autosampler 32 is a device configured to obtain a synthesized sample from the nucleic acid synthesizing device 31. The spectrophotometer 33 is a device configured to measure absorbance from the sample obtained by the autosampler 32. Data indicating absorbance measured by the spectrophotometer 33 is input to the condition optimization device 10 as an actual reaction value.

The overall configuration of the condition optimization system 1 illustrated in FIG. 1 is merely illustrative, and various system configuration examples are possible in accordance with applications and purposes. For example, the condition optimization device 10 may be realized by a plurality of computers or may be realized as a cloud computing service. Further, for example, the condition optimization system 1 may be realized by a stand-alone information processing device having the functions of both the condition optimization device 10 and the user terminal 20.

<Hardware configuration of condition optimization system>

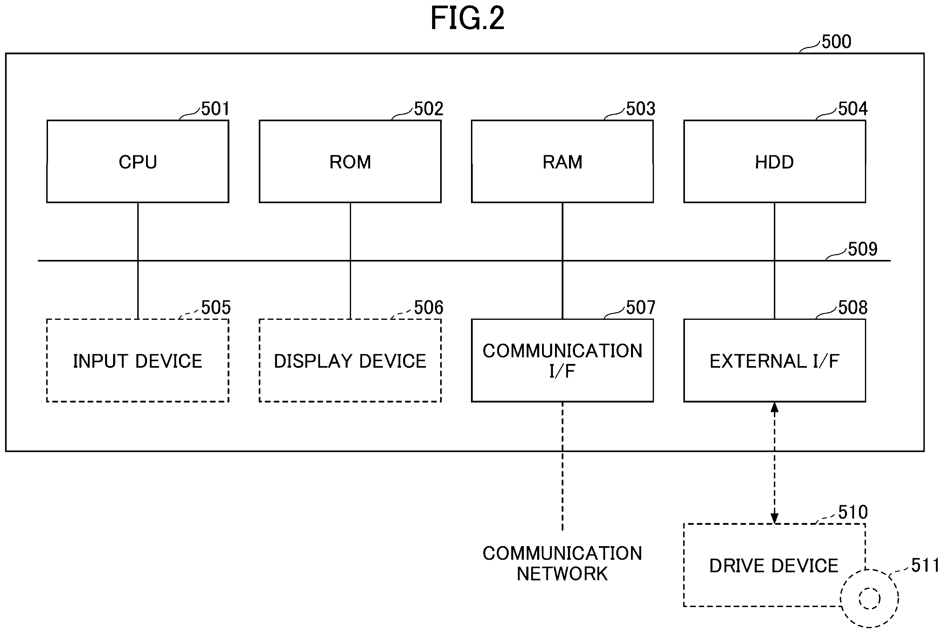

Next, the hardware configuration of the condition optimization system according to the present embodiment will be described with reference to FIG. 2.

<<Hardware configuration of computer>>

The condition optimization device 10 and the user terminal 20 according to the present embodiment are realized, for example, by a computer. FIG. 2 is a block diagram illustrating an example of the hardware configuration of a computer 500 according to the present embodiment.

As illustrated in FIG. 2, the computer 500 includes a Central Processing Unit (CPU) 501, a Read Only Memory (ROM) 502, a Random Access Memory (RAM) 503, a Hard Disk Drive (HDD) 504, an input device 505, a display device 506, a communication interface (I/F) 507, and an external I/F 508. The CPU 501, the ROM 502, and the RAM 503 form what is generally referred to as a computer. The respective hardware pieces of the computer 500 are mutually connected through a bus line 509. The input device 505 and the display device 506 may be used while being connected to the external I/F 508.

The CPU 501 is an operating device configured to realize controls and functions of the entirety of the computer 500 by reading out programs and data onto the RAM 503 from a memory device, such as the ROM 502, the HDD 504, or the like, and executing processes.

The ROM 502 is an example of a nonvolatile semiconductor memory (memory device) that can retain programs and data even when power supply is cut. The ROM 502 functions as a main memory device configured to store, for example, various programs and data necessary for the CPU 501 to execute various programs installed on the HDD 504. Specifically, the ROM 502 stores boot programs, such as Basic Input/Output System (BIOS), Extensible Firmware Interface (EFI), and the like, that are executed when the computer 500 is started, and data, such as Operating System (OS) settings, network settings, and the like.

The RAM 503 is an example of a volatile semiconductor memory (memory device) from which programs and data are erased when power supply is cut. The RAM 503 is, for example, a Dynamic Random Access Memory (DRAM), a Static Random Access Memory (SRAM), or the like. The RAM 503 provides a work area in which various programs installed on the HDD 504 are deployed when executed by the CPU 501.

The HDD 504 is an example of a nonvolatile memory device storing programs and data. The programs and data stored in the HDD 504 include OS, which is basic software for controlling the entirety of the computer 500, and applications that provide various functions on the OS. The computer 500 may employ a memory device using a flash memory as a memory medium (e.g., a Solid State Drive (SSD) or the like), instead of the HDD 504.

The input device 505 is, for example, a touch panel, operation keys or buttons, or a keyboard or a mouse by which a user enters various signals, or a microphone by which a user enters sound data, such as sound and voice.

The display device 506 is formed by a liquid crystal or organic Electro-Luminescence (EL) display configured to display a screen, and a loudspeaker configured to output sound data, such as sound and voice.

The communication I/F 507 is an interface configured to connect to a communication network in order that the computer 500 can perform data communication.

The external I/F 508 is an interface to an external device. An example of the external device is a drive device 510 or the like.

The drive device 510 is a device configured for a recording medium 511 to be set therein. The recording medium 511 meant here encompasses media configured to record information optically, electrically, or magnetically, such as a CD-ROM, a flexible disk, and a magneto-optical disk. The recording medium 511 may also encompass, for example, semiconductor memories configured to record information electrically, such as a ROM, a flash memory, and the like. Hence, the computer 500 can perform either or both of reading from and writing into the recording medium 511 via the external I/F 508.

Various programs to be installed on the HDD 504 are installed, for example, by a distributed recording medium 511 being set in the drive device 510 connected to the external I/F 508, and various programs recorded in the recording medium 511 being read out by the drive device 510. Alternatively, various programs to be installed on the HDD 504 may be installed by being downloaded from any other network different from the communication network via the communication I/F 507.

<Functional configuration of condition optimization system>

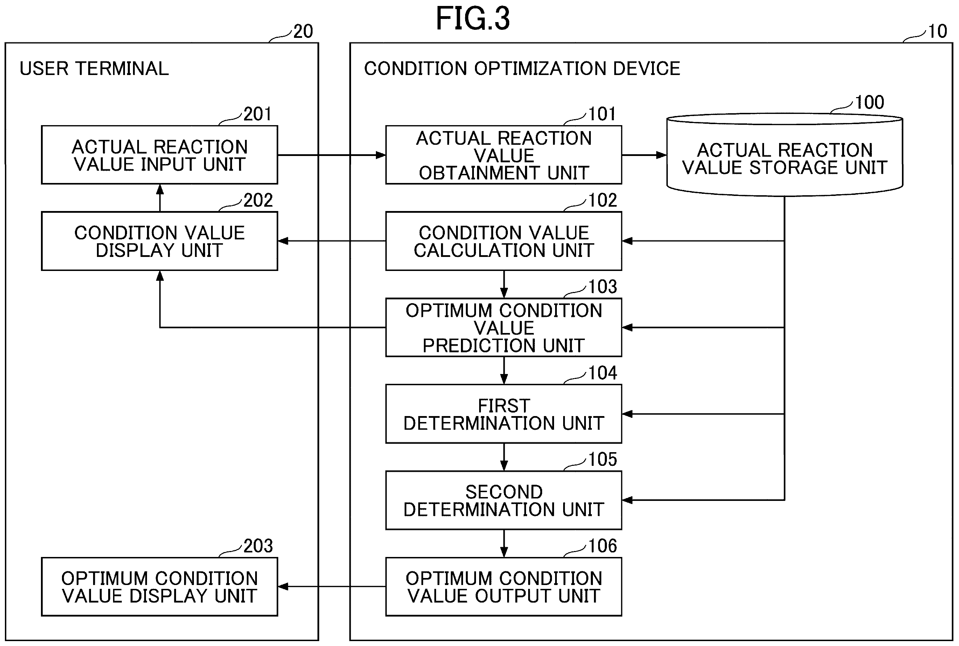

Next, the functional configuration of the condition optimization system according to the present embodiment will be described with reference to FIG. 3. FIG. 3 is a block diagram illustrating an example of the functional configuration of the condition optimization system according to the present embodiment.

<<Functional configuration of condition optimization device>>

As illustrated in FIG. 3, the condition optimization device 10 according to the present embodiment includes an actual reaction value storage unit 100, an actual reaction value obtainment unit 101, a condition value calculation unit 102, an optimum condition value prediction unit 103, a first determination unit 104, a second determination unit 105, and an optimum condition value output unit 106.

The actual reaction value storage unit 100 is realized using, for example, the HDD 504 illustrated in FIG. 2. The actual reaction value obtainment unit 101, the condition value calculation unit 102, the optimum condition value prediction unit 103, the first determination unit 104, the second determination unit 105, and the optimum condition value output unit 106 are realized through processes executed by the CPU 501 in accordance with the programs deployed on the RAM 503 from the HDD 504 illustrated in FIG. 2.

The actual reaction value storage unit 100 is configured to accumulate the actual reaction value received from the user terminal 20. The condition value used upon obtaining the actual reaction value is associated with this actual reaction value. That is, the actual reaction value storage unit 100 stores a plurality of condition values and actual reaction values that were used and obtained in the past. In the following, the newest condition value stored in the actual reaction value storage unit 100 is also referred to as "previous condition value".

The actual reaction value obtainment unit 101 is configured to receive the actual reaction value from the user terminal 20. Also, the actual reaction value obtainment unit 101 is configured to associate the received actual reaction value with the condition value and store the associated actual reaction value in the actual reaction value storage unit 100.

The condition value calculation unit 102 is configured to calculate a condition value to be experimented next based on the actual reaction value accumulated in the actual reaction value storage unit 100. Bayesian optimization is used for calculation of the condition value. In the following, the condition value calculated the most recently by the condition value calculation unit 102 is also referred to as "current condition value".

The optimum condition value prediction unit 103 is configured to predict, based on the actual reaction value accumulated in the actual reaction value storage unit 100, the best observable actual reaction value (hereinafter also referred to as "best physical property value") and a condition value at which the best physical property value is obtainable (hereinafter also referred to as "optimum condition value"). The response surface method is used for prediction of the best physical property value and the optimum condition value.

The first determination unit 104 is configured to determine whether or not the condition value has converged based on the condition value calculated by the condition value calculation unit 102 and the condition value stored in the actual reaction value storage unit 100.

The first determination unit 104 according to the present embodiment is configured to determine whether or not the condition value has converged based on the Euclidean distance between the current condition value and the previous condition value. That is, the first determination unit 104 calculates the Euclidean distance between each of the variables included in the condition value calculated by the optimum condition value prediction unit 103 and each of the variables included in the newest condition value stored in the actual reaction value storage unit 100. The first determination unit 104 compares the calculated Euclidean distance with a predetermined threshold, thereby determining whether or not the condition value has converged.

When the first determination unit 104 determines that the condition value has converged, the second determination unit 105 is configured to determine whether or not the condition value has converged based on the best physical property value and the actual reaction value observed in accordance with the optimum condition value predicted by the optimum condition value prediction unit 103 (hereinafter this actual reaction value is also referred to as "target physical property value").

The second determination unit 105 according to the present embodiment is configured to determine whether or not the condition value has converged based on the difference between the target physical property value and the best physical property value. That is, the second determination unit 105 calculates the difference between the best physical property value predicted by the optimum condition value prediction unit 103 and the target physical property value obtained by the actual reaction value obtainment unit 101. The second determination unit 105 compares the calculated difference with a predetermined threshold, thereby determining whether or not the condition value has converged.

When the second determination unit 105 determines that the condition value has converged, the optimum condition value output unit 106 is configured to transmit, to the user terminal 20, the optimum condition value predicted the most recently by the optimum condition value prediction unit 103. In the following, the optimum condition value predicted the most recently by the optimum condition value prediction unit 103 is also referred to as "current optimum condition value".

<<Functional configuration of user terminal>>

As illustrated in FIG. 3, the user terminal 20 according to the present embodiment includes an actual reaction value input unit 201, a condition value display unit 202, and an optimum condition value display unit 203.

The actual reaction value input unit 201 is realized through a process executed by the CPU 501 and the input device 505 in accordance with the programs deployed on the RAM 503 from the HDD 504 illustrated in FIG. 2. The condition value display unit 202 and the optimum condition value display unit 203 are realized through processes executed by the CPU 501 and the display device 506 in accordance with the programs deployed on the RAM 503 from the HDD 504 illustrated in FIG. 2.

The actual reaction value input unit 201 is configured to receive an input of an actual reaction value in response to a user's operation. Also, the actual reaction value input unit 201 is configured to transmit the received actual reaction value to the condition optimization device 10.

The condition value display unit 202 is configured to receive the condition value from the condition optimization device 10. Also, the condition value display unit 202 is configured to display the received condition value on the display device 506.

The optimum condition value display unit 203 is configured to receive the optimum condition value from the condition optimization device 10. Also, the optimum condition value display unit 203 is configured to display the received optimum condition value on the display device 506.

<Process of condition optimization method>

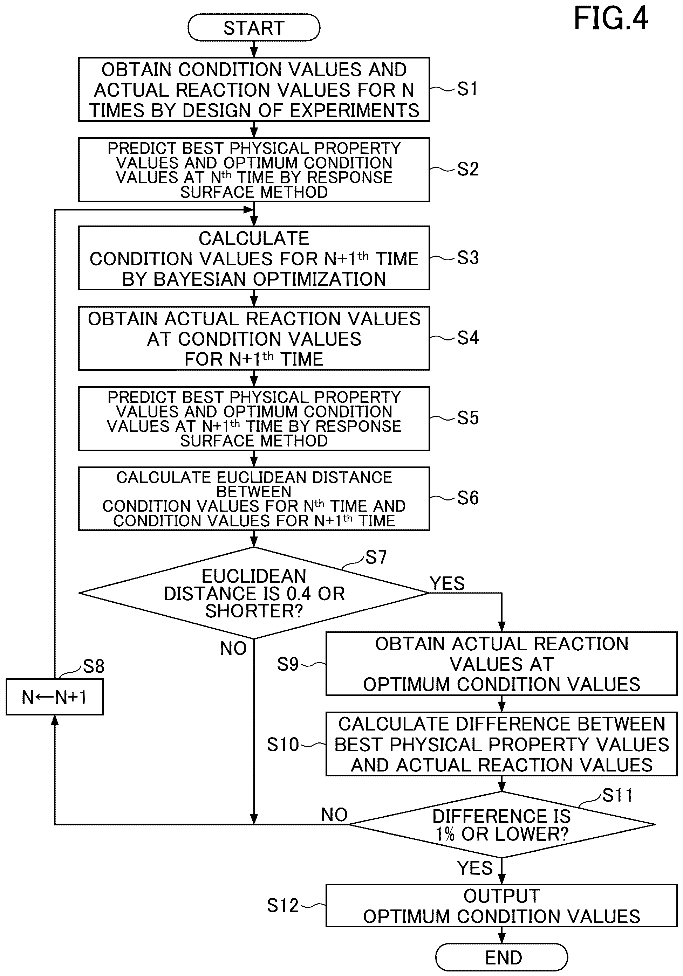

Next, the process of the condition optimization method executed by the condition optimization system according to the present embodiment will be described with reference to FIG. 4. FIG. 4 is a flowchart illustrating an example of the process of the condition optimization method according to the present embodiment.

In step S1, the actual reaction value input unit 201 receives an input of actual reaction values for N times by design of experiments in response to a user's operation. Next, the actual reaction value input unit 201 transmits the received actual reaction values for N times to the condition optimization device 10.

In the condition optimization device 10, the actual reaction value obtainment unit 101 receives the actual reaction values for N times from the user terminal 20. Next, the actual reaction value obtainment unit 101 stores the received actual reaction values for N times in the actual reaction value storage unit 100.

According to Bayesian optimization, there should be initial data of the actual reaction values, and desirably, the initial data is combinations of the condition values obtained in a wide range. Therefore, in the present embodiment, actual reaction values serving as the initial data are obtained by design of experiments.

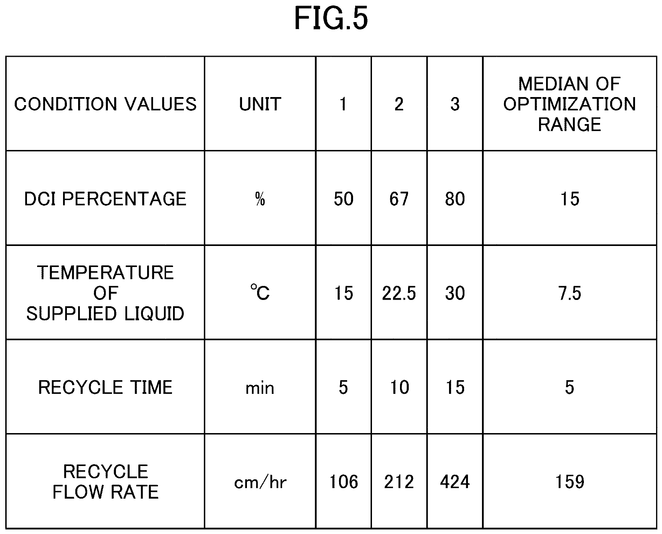

In the present embodiment, assuming measurement of a reaction rate in the synthesis of DNA oligonucleotides, an L9 orthogonal array of 3 levels and 4 factors is used for input of actual reaction values for 9 times (i.e., N = 9). FIG. 5 illustrates an example of the L9 orthogonal array. As illustrated in FIG. 5, in the present embodiment, the 4 factors that are DCI percentage, temperature of supplied liquid, recycle time, and recycle flow rate are each changed to 3 levels illustrated as 1 to 3, i.e., the condition values for 9 times are used, thereby obtaining reaction rates observed at the condition values as actual reaction values. However, the condition values and the actual reaction values in the present embodiment are not limited thereto. Any experiment may be used as long as predetermined physical property values can be obtained at predetermined condition values.

For example, in the case of nucleic acid synthesis, first, deprotection waste liquid after synthesis in the nucleic acid synthesizing device 31 is recovered by the autosampler 32 (available from Gilson Inc.). In the autosampler 32, the recovered deprotection waste liquid is automatically diluted with a solution of 0.1M paratoluenesulfonic acid monohydrate (pTSA) in acetonitrile. By using the spectrophotometer 33 (available from BAS Co., Ltd.), it is possible to automatically obtain data of actual reaction values (absorbance) from the deprotection group (DMTr) included in the deprotection waste liquid. With this configuration, experiments can be performed continuously and efficiently without the intervention of an experimenter.

As illustrated in FIG. 4, in step S2, the optimum condition value prediction unit 103 predicts, by the response surface method, the best physical property value and the optimum condition value based on the actual reaction values for N times stored in the actual reaction value storage unit 100. The best physical property value is the best observable actual reaction value (here, the maximum reaction rate). The optimum condition value is the condition value at which obtainment of the maximum reaction rate at the $N^{th}$ time (N = 9) is predicted. Next, the optimum condition value prediction unit 103 transmits the predicted best physical property value to the second determination unit 105.

In step S3, the condition value calculation unit 102 calculates, by Bayesian optimization, condition values at the $N+1^{th}$ time based on the actual reaction values for the N times accumulated in the actual reaction value storage unit 100. Next, the condition value calculation unit 102 transmits the calculated condition values at the $N+1^{th}$ time to the first determination unit 104.

In step S4, the condition value calculation unit 102 transmits the condition values at the $N+1^{th}$ time calculated in step S3 to the user terminal 20. In the user terminal 20, the condition value display unit 202 receives the condition values at the $N+1^{th}$ time from the condition optimization device 10. Next, the condition value display unit 202 displays the received condition values at the N+1$^{th}$ time on the display device 506.

The user performs the N+1$^{th}$ experiment in accordance with the condition values at the N+1$^{th}$ time displayed on the display device 506 of the user terminal 20. When the N+1$^{th}$ experiment is completed and actual reaction values are obtained, the user inputs the actual reaction values at the N+1$^{th}$ time to the user terminal 20. The actual reaction value input unit 201 receives an input of the actual reaction values at the N+1$^{th}$ time, and transmits the received actual reaction values to the condition optimization device 10. For example, like in step S1, if the deprotection waste liquid after nucleic acid synthesis is recovered and diluted by the autosampler 32 and the data of the actual reaction values can be automatically obtained by the spectrophotometer 33, the experiment can be performed continuously and efficiently.

In the condition optimization device 10, the actual reaction value obtainment unit 101 receives the actual reaction values at the N+1$^{th}$ time from the user terminal 20. Next, the actual reaction value obtainment unit 101 associates the received actual reaction values at the N+1$^{th}$ time with the condition values at the N+1$^{th}$ time, and stores the associated actual reaction values in the actual reaction value storage unit 100.

In step S5, the optimum condition value prediction unit 103 predicts, by the response surface method, the best physical property value and the optimum condition value based on the actual reaction values for the N+1 times accumulated in the actual reaction value storage unit 100. The best physical property value is the best observable actual reaction value (here, the maximum reaction rate). The optimum condition value is the condition value at which obtainment of the maximum reaction rate at the N+1$^{th}$ time (N = 10) is predicted. Next, the optimum condition value prediction unit 103 transmits the predicted best physical property value to the second determination unit 105.

In step S6, the first determination unit 104 receives the condition values at the N$^{th}$ time and the N+1$^{th}$ time from the condition value calculation unit 102. Next, the first determination unit 104 calculates the Euclidean distance between the condition value at the N+1$^{th}$ time and the condition value at the N$^{th}$ time.

Specifically, the first determination unit 104 calculates the Euclidean distance d(x,y), where x is the condition value at the N+1$^{th}$ time, and y is the condition value at the N$^{th}$ time in accordance with the following formula.

[Formula 1]

$$d(x, y) = \sqrt{\left(\frac{x_1 - y_1}{A_1}\right)^2 + \cdots + \left(\frac{x_n - y_n}{A_n}\right)^2}$$

In this formula, $x_1$, ..., $x_n$ are each of the variables included in the condition values at the N+1$^{th}$ time, $y_1$, ..., $y_n$ are each of the variables included in the condition values at the N$^{th}$ time, and $A_1$, ..., $A_n$ are a value 1/2 the difference between the upper and lower limits of each of the variables ($A_n = (Max_n - Min_n)/2$).

In step S7, the first determination unit 104 compares the Euclidean distance calculated in step S6 with a predetermined threshold, thereby determining whether or not the condition value has converged. Specifically, when the calculated Euclidean distance is 0.4 or shorter, it is determined that convergence has occurred.

When the first determination unit 104 determines that convergence has occurred (YES), the first determination unit 104 causes the process to proceed to step S9. Meanwhile, when the first determination unit 104 determines that convergence has not occurred (NO), the first determination unit 104 causes the process to proceed to step S8.

In step S8, the condition optimization device 10 increments N, i.e., calculates N←N+1. Subsequently, the process of from step S3 through step S7 is performed again.

In step S9, the optimum condition value prediction unit 103 transmits the optimum condition value calculated in step S2 to the user terminal 20. In the user terminal 20, the condition value display unit 202 receives the optimum condition value from the condition optimization device 10. Next, the condition value display unit 202 displays the received optimum condition value on the display device 506.

The user performs an experiment in accordance with the optimum condition value displayed on the display device 506 of the user terminal 20. When the experiment is completed and actual reaction values (i.e., the target physical property value) are obtained, the user inputs the target physical property value to the user terminal 20. The actual reaction value input unit 201 receives an input of the target physical property value, and transmits the received target physical property value to the condition optimization device 10.

In the condition optimization device 10, the actual reaction value obtainment unit 101 receives the target physical property value from the user terminal 20. Next, the actual reaction value obtainment unit 101 transmits the received target physical property value to the second determination unit 105.

In step S10, the second determination unit 105 receives the best physical property value from the optimum condition value prediction unit 103. Next, the second determination unit 105 receives the target physical property value from the actual reaction value obtainment unit 101. Next, the second determination unit 105 calculates the difference between the best physical property value and the target physical property value.

In step S11, the second determination unit 105 compares the difference calculated in step S10 with a predetermined threshold, thereby determining whether or not the condition value has converged. Specifically, it is determined that convergence has occurred when the calculated difference is 1% or lower with respect to the best physical property value.

When the second determination unit 105 determines that convergence has occurred (YES), the second determination unit 105 causes the process to proceed to step S12. Meanwhile, when the second determination unit 105 determines that convergence has not occurred (NO), the second determination unit 105 causes the process to proceed to step S8.

In step S12, the optimum condition value output unit 106 transmits, to the user terminal 20, the optimum condition value predicted in the most-recently executed step S2 (i.e., the current optimum condition value). In the user terminal 20, the optimum condition value display unit 203 receives the optimum condition value from the condition optimization device 10. Next, the optimum condition value display unit 203 displays the received optimum condition value on the display device 506.

<Effects of first embodiment>

The condition optimization system according to the present embodiment determines whether or not the condition value calculated by Bayesian optimization has converged by utilizing the response surface method. The response surface method can predict the best actual reaction value with a small number of experiments. The condition optimization system according to the present embodiment determines that the condition value has converged when the prediction accuracy by the response surface method is sufficiently high. Therefore, according to the condition optimization system of the present embodiment, it is possible to detect that the condition value has converged with a small number of experiments by utilizing the response surface method.

Moreover, according to the condition optimization system according to the present embodiment, an appropriate threshold for determining convergence is set by analyzing various actual reaction values. It is found that convergence can be determined with high accuracy when the Euclidean distance between the condition values is 0.4 or shorter and the difference between the predicted best physical property value and the actually measured physical property value is 1% or lower.

Therefore, according to the condition optimization system according to the present embodiment, it is possible to determine whether or not the condition value calculated by Bayesian optimization has converged with a small number of experiments. That is, according to the condition optimization system of the present embodiment, it is possible to efficiently optimize the conditions.

[Second embodiment]

In the first embodiment, the first determination unit 104 is configured to determine whether or not the condition value has converged based on the Euclidean distance between the current condition value and the previous condition value. In the second embodiment, the first determination unit 104 is configured to determine whether or not the condition value has converged based on the standard deviation of the current condition value and the most-recent condition values.

In the following, the condition optimization system 1 according to the present embodiment will be described by focusing on the differences from the condition optimization system 1 according to the first embodiment.

<Process of condition optimization method>

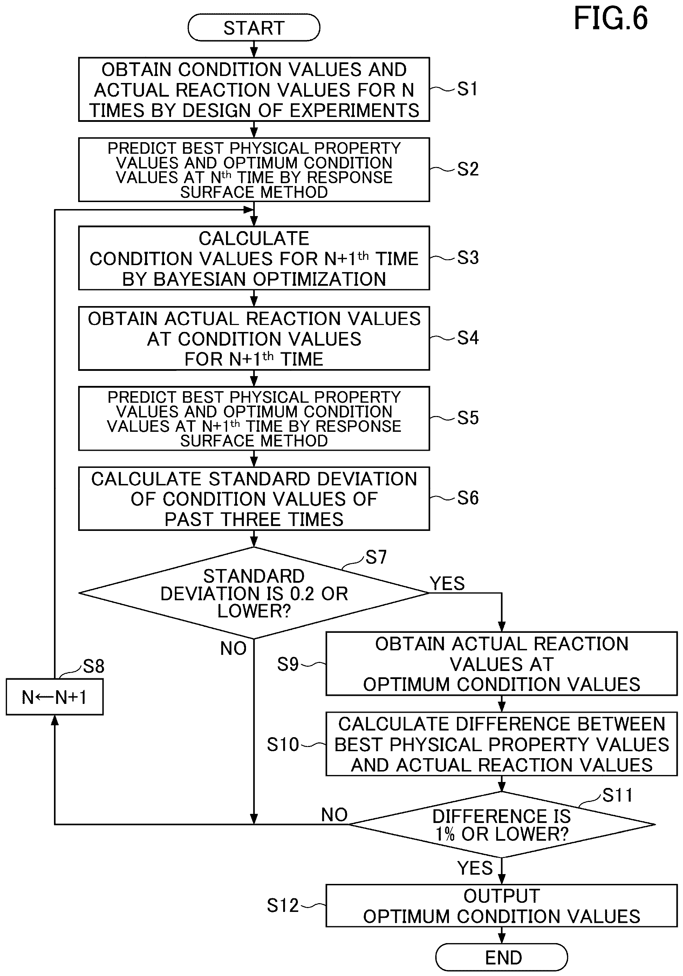

The process of the condition optimization method executed by the condition optimization system according to the present embodiment will be described with reference to FIG. 6. FIG. 6 is a flowchart illustrating an example of the process of the condition optimization method according to the present embodiment.

In the following, the condition optimization method according to the present embodiment will be described by focusing on the differences from the first embodiment.

In step S6, the first determination unit 104 according to the present embodiment determines whether or not the condition value has converged based on the standard deviation of the condition values for the most-recent three times. That is, the first determination unit 104 calculates the standard deviation of: each of the variables included in the condition values calculated by the condition value calculation unit 102; and each of the variables included in the condition values for the most-recent two times stored in the actual reaction value storage unit 100.

Specifically, the first determination unit 104 calculates the standard deviation as follows: (1) obtaining a value by dividing the value of a variable included in the condition value at the $N^{th}$ time by the median of an optimization range of the variable, where the optimization range is a range between the upper limit and the lower limit of the values that can be taken by that variable as an experimental condition; (2) obtaining a value by dividing the value of the variable included in the condition value at the $N-1^{th}$ time by the median of the optimization range of the variable; (3) obtaining a value by dividing the value of the variable included in the condition value at the $N-2^{th}$ time by the median of the optimization range of the variable; (4) performing the above (1) to (3) for all variables; and (4) calculating the standard deviation of the values calculated in the above (1) to (3) for all variables included in the condition value.

Here, the example of calculating the standard deviation of the most-recent three times is described. However, the number of condition values for calculating the standard deviation is not limited. The standard deviation of the most-recent four or more times may be calculated.

In step S7, the first determination unit 104 according to the present embodiment compares each of the standard deviations of each variable calculated in step S6 with a predetermined threshold, thereby determining whether or not the condition value has converged. Specifically, when all of the calculated standard deviations are 0.2 or lower, it is determined that convergence has occurred.

<Effects of second embodiment>

The condition optimization system according to the present embodiment determines whether or not the condition value calculated by Bayesian optimization has converged based on the standard deviation of each of the variables included in the condition value. At this time, an appropriate threshold for determining convergence is set by analyzing various actual reaction values. It is found that convergence can be determined with high accuracy when all of the standard deviations of each variable included in the condition value are 0.2 or lower.

Therefore, according to the condition optimization system according to the present embodiment, it is possible to determine whether or not the condition value calculated by Bayesian optimization has converged with a small number of experiments. That is, according to the condition optimization system according to the present embodiment, it is possible to efficiently optimize the conditions.

[Examples]

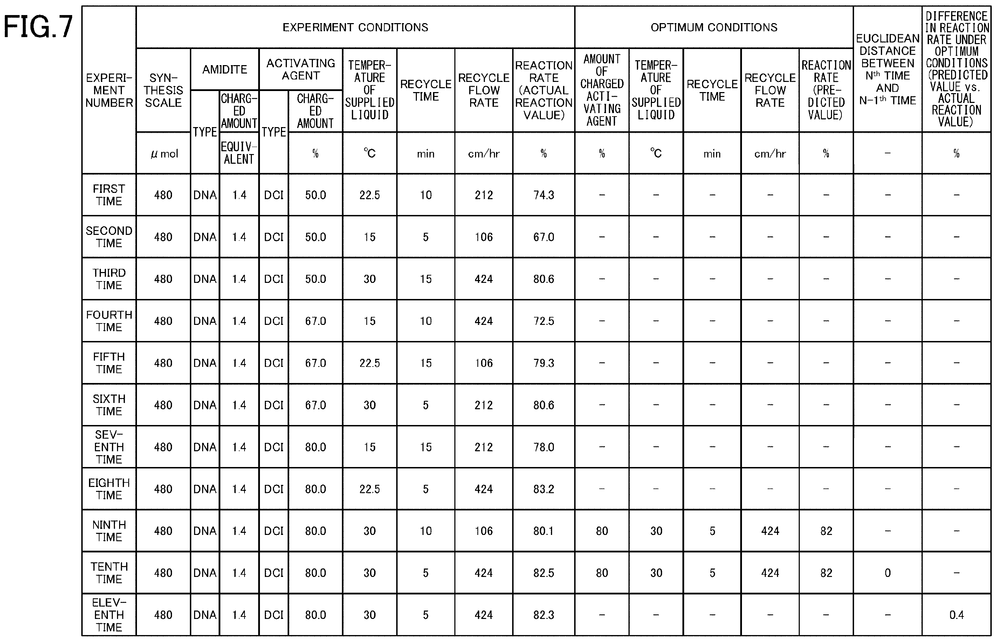

An example in which the condition optimization system according to the first embodiment is applied to the synthesis of DNA oligonucleotides will be described with reference to FIG. 7. FIG. 7 is a table illustrating actual reaction values according to the present embodiment.

Porous resin beads were charged into a synthesis column (volume: 12.6 ml) so as to be of a synthesis scale of 480 pmol. The synthesis column was set in an oligonucleotide automatic synthesizing device. Nucleoside phosphoroamidite and 4,5-dicyanoimidazole (DCI), serving as an activating agent, were added thereto in the corresponding amounts described in FIG. 7, followed by performing a coupling reaction under the conditions described in FIG. 7.

The activating agent was dissolved in acetonitrile and prepared to have a concentration of 0.7M. Other synthesis reagents used were: 3% DCA in toluene as a deprotecting agent; a 0.05M oxidizing solution for the above synthesis device, serving as an oxidant; a mixed solution of lutidine, N-methylimidazole, and acetic anhydride in acetonitrile, serving as a capping agent; and TBA in acetonitrile (at a ratio of 8:2), serving as an amine wash reaction solution. Under a DMT-off condition, 24 mer DNA oligonucleotides (5'-TCGACGTATTGACGTATTGACGTA-3', all oxidized: SEQ ID NO: 1) were synthesized.

After completion of the synthesis, by immersing the dried DNA oligonucleotide-bound porous resin beads in aqueous ammonia, the DNA oligonucleotides were cut out from the porous resin beads. Then, deprotection of the amino groups of the bases was performed, and a filtrate including the DNA oligonucleotides dissolved therein was obtained.

The reaction rate was measured as follows. DNA is synthesized 1 mer by 1 mer through the steps of deprotection, coupling, oxidation, and capping. Then, the reaction rate was evaluated by recovering all of the deprotection waste liquid of each mer and quantifying the deprotection group (DMTr) included therein. The deprotection rate was calculated through HPLC measurement after appropriate dilution with an acetonitrile solution of 0.1M para-toluenesulfonic acid monohydrate (pTSA).

The reaction rate was calculated by dividing the absolute amount of the DMTr, obtained from the above measurement, by the total reaction point (= reaction point of the beads (pmol/g) $\times$ weight of the beads).

It is appropriate to obtain a wide range of combinations of condition values as the initial data for condition optimization. Therefore, synthesis was performed under the L9 condition in accordance with the orthogonal array of 3 levels and 4 factors illustrated in FIG. 5, and nine initial data was obtained (first to ninth times in FIG. 7).

Based on the initial data, the condition value to be experimented next was calculated by Bayesian optimization. Synthesis was performed at the calculated condition value, and the reaction rate was obtained in the same manner. The obtained result was added to the initial data, the condition value to be experimented next was calculated again by Bayesian optimization, synthesis was performed at the calculated condition value ...; this process was performed repeatedly. Prediction by the response surface method was performed at the same time as Bayesian optimization, and the maximum reaction rate predicted for each of the syntheses was obtained.

As illustrated in FIG. 7, in the tenth experiment, the Euclidean distance between the condition value at the $N^{th}$ time and the condition value at the $N\text{-}1^{th}$ time is 0.4 or lower. In the eleventh experiment, the difference between the reaction rate (actual reaction value) and the reaction rate (predicted value) is 1% or lower. Therefore, in the present embodiment, it is determined that the condition value has converged at the tenth time. Moreover, according to the actual reaction value, it can be found that the optimum condition value is obtained at the tenth time.

As described above, in the present embodiment, it is indicated that the condition optimization system according to the first embodiment can optimize the condition value with a small number of experiments.

[Supplemental]

Each function of the embodiments described above can be realized by one or a plurality of processing circuits. In the present specification, a "processing circuit" encompasses devices such as a processor programmed by software to execute each function, such as a processor implemented on an electronic circuit, and an Application Specific Integrated Circuit (ASIC), a Digital Signal Processor (DSP), a Field Programmable Gate Array (FPGA), and existing circuit modules that are designed to execute each function described above.

The embodiments of the present invention have been described above in detail. However, the present invention is not limited to these embodiments, and various modifications or changes can be applied to the present invention within the scope of the spirit of the present invention described in the claims.

The present application claims priority to Japanese Patent Application No. 2022-25318 filed with the Japan Patent Office on February 22, 2022, the entire contents of which are incorporated herein by reference.

REFERENCE SIGNS LIST

1     Condition optimization system
10    Condition optimization device
101   Actual reaction value obtainment unit
102   Condition value calculation unit
103   Optimum condition value prediction unit
104   First determination unit
105   Second determination unit
106   Optimum condition value output unit
20    User terminal
201   Actual reaction value input unit
202   Condition value display unit
203   Optimum condition value display unit

**Claims**

1. A condition optimization device, comprising:

   an actual reaction value obtainment unit configured to obtain an actual reaction value by observing a predetermined physical property value at a predetermined condition value;
   a condition value calculation unit configured to calculate a new condition value by Bayesian optimization;
   an optimum condition value prediction unit configured to predict, by a response surface method, a best physical property value and an optimum condition value at which the best physical property value is obtainable;
   a first determination unit configured to determine whether or not convergence has occurred based on the condition value corresponding to the actual reaction value and based on the new condition value; and
   a second determination unit configured to determine, upon the first determination unit determining that the convergence has occurred, whether or not convergence has occurred based on a difference between the physical property value observed at the optimum condition value and the best physical property value.

2. The condition optimization device as claimed in claim 1, wherein

the first determination unit is configured to determine whether or not the convergence has occurred, by comparing a Euclidean distance with a predetermined threshold, the Euclidean distance being between: the condition value corresponding to the actual reaction value; and the new condition value.

3. The condition optimization device as claimed in claim 2, wherein
the first determination unit is configured to determine that the convergence has occurred upon the Euclidean distance being 0.4 or shorter.

4. The condition optimization device as claimed in claim 1, wherein
the first determination unit is configured to determine whether or not the convergence has occurred, by comparing a standard deviation with a predetermined threshold, the standard deviation being of: a plurality of condition values each being the condition value corresponding to the actual reaction value; and the new condition value.

5. The condition optimization device as claimed in claim 4, wherein
the first determination unit is configured to determine that the convergence has occurred upon the standard deviation being 0.2 or lower.

6. The condition optimization device as claimed in any one of claims 1 to 5, wherein
the second determination unit is configured to determine that the convergence has occurred upon a difference being 1% or lower, the difference being between the physical property value observed at the optimum condition value and the best physical property value.

7. The condition optimization device as claimed in any one of claims 1 to 6, wherein
the actual reaction value is a value that is obtained by automatically obtaining a sample after actual reaction and automatically analyzing the sample with a spectrophotometer.

8. A condition optimization method, comprising:

an actual reaction value obtainment procedure of obtaining an actual reaction value by observing a predetermined physical property value at a predetermined condition value;
a condition value calculation procedure of calculating a new condition value by Bayesian optimization;
an optimum condition value prediction procedure of predicting, by a response surface method, a best physical property value and an optimum condition value at which the best physical property value is obtainable;
a first determination procedure of determining whether or not convergence has occurred based on the condition value corresponding to the actual reaction value and based on the new condition value; and
a second determination procedure of determining whether or not convergence has occurred based on a difference between the physical property value observed at the optimum condition value and the best physical property value upon occurrence of the convergence being determined in the first determination procedure,
the actual reaction value obtainment procedure, the condition value calculation procedure, the optimum condition value prediction procedure, the first determination procedure, and the second determination procedure being executed by a computer.

9. A program causing a computer to execute:

an actual reaction value obtainment procedure of obtaining an actual reaction value by observing a predetermined physical property value at a predetermined condition value;
a condition value calculation procedure of calculating a new condition value by Bayesian optimization;
an optimum condition value prediction procedure of predicting, by a response surface method, a best physical property value and an optimum condition value at which the best physical property value is obtainable;
a first determination procedure of determining whether or not convergence has occurred based on the condition value corresponding to the actual reaction value and based on the new condition value; and
a second determination procedure of determining whether or not convergence has occurred based on a difference between the physical property value observed at the optimum condition value and the best physical property value upon occurrence of the convergence being determined in the first determination procedure.

# FIG.1

# FIG.2

EP 4 485 298 A1

# FIG.3

**FIG.4**

START

OBTAIN CONDITION VALUES AND ACTUAL REACTION VALUES FOR N TIMES BY DESIGN OF EXPERIMENTS —S1

PREDICT BEST PHYSICAL PROPERTY VALUES AND OPTIMUM CONDITION VALUES AT $N^{th}$ TIME BY RESPONSE SURFACE METHOD —S2

CALCULATE CONDITION VALUES FOR $N+1^{th}$ TIME BY BAYESIAN OPTIMIZATION —S3

OBTAIN ACTUAL REACTION VALUES AT CONDITION VALUES FOR $N+1^{th}$ TIME —S4

PREDICT BEST PHYSICAL PROPERTY VALUES AND OPTIMUM CONDITION VALUES AT $N+1^{th}$ TIME BY RESPONSE SURFACE METHOD —S5

CALCULATE EUCLIDEAN DISTANCE BETWEEN CONDITION VALUES FOR $N^{th}$ TIME AND CONDITION VALUES FOR $N+1^{th}$ TIME —S6

S7
EUCLIDEAN DISTANCE IS 0.4 OR SHORTER?

YES

NO

S8
N←N+1

S9
OBTAIN ACTUAL REACTION VALUES AT OPTIMUM CONDITION VALUES

S10
CALCULATE DIFFERENCE BETWEEN BEST PHYSICAL PROPERTY VALUES AND ACTUAL REACTION VALUES

S11
NO    DIFFERENCE IS 1% OR LOWER?

YES

S12
OUTPUT OPTIMUM CONDITION VALUES

END

# FIG.5

| CONDITION VALUES | UNIT | 1 | 2 | 3 | MEDIAN OF OPTIMIZATION RANGE |
|---|---|---|---|---|---|
| DCI PERCENTAGE | % | 50 | 67 | 80 | 15 |
| TEMPERATURE OF SUPPLIED LIQUID | °C | 15 | 22.5 | 30 | 7.5 |
| RECYCLE TIME | min | 5 | 10 | 15 | 5 |
| RECYCLE FLOW RATE | cm/hr | 106 | 212 | 424 | 159 |

FIG.6

```
                    ┌─────────────┐
                    │    START    │
                    └──────┬──────┘
                           │
        ┌──────────────────┴──────────────────┐
        │ OBTAIN CONDITION VALUES AND         │
        │ ACTUAL REACTION VALUES FOR N        │──S1
        │ TIMES BY DESIGN OF EXPERIMENTS      │
        └──────────────────┬──────────────────┘
                           │
        ┌──────────────────┴──────────────────┐
        │ PREDICT BEST PHYSICAL PROPERTY      │
        │ VALUES AND OPTIMUM CONDITION        │──S2
        │ VALUES AT Nth TIME BY RESPONSE      │
        │ SURFACE METHOD                      │
        └──────────────────┬──────────────────┘
                           │
        ┌──────────────────┴──────────────────┐
        │ CALCULATE                           │
        │ CONDITION VALUES FOR N+1th TIME     │──S3
        │ BY BAYESIAN OPTIMIZATION            │
        └──────────────────┬──────────────────┘
                           │
        ┌──────────────────┴──────────────────┐
        │ OBTAIN ACTUAL REACTION VALUES       │
        │ AT CONDITION VALUES                 │──S4
        │ FOR N+1th TIME                      │
        └──────────────────┬──────────────────┘
                           │
        ┌──────────────────┴──────────────────┐
        │ PREDICT BEST PHYSICAL PROPERTY      │
        │ VALUES AND OPTIMUM CONDITION        │──S5
        │ VALUES AT N+1th TIME BY RESPONSE    │
        │ SURFACE METHOD                      │
        └──────────────────┬──────────────────┘
                           │
        ┌──────────────────┴──────────────────┐
        │ CALCULATE STANDARD DEVIATION        │
        │ OF CONDITION VALUES OF              │──S6
        │ PAST THREE TIMES                    │
        └──────────────────┬──────────────────┘
                           │
                   ╱───────┴───────╲  S7
                  ╱  STANDARD       ╲      YES
                 ╱ DEVIATION IS 0.2  ╲────────────┐
                 ╲    OR LOWER?      ╱            │
                  ╲                 ╱             │
                   ╲───────┬───────╱              │
                        NO │                      │
```

S8  N←N+1

S9 ─ OBTAIN ACTUAL REACTION VALUES AT OPTIMUM CONDITION VALUES

S10 ─ CALCULATE DIFFERENCE BETWEEN BEST PHYSICAL PROPERTY VALUES AND ACTUAL REACTION VALUES

S11  DIFFERENCE IS 1% OR LOWER?  NO

YES

S12 ─ OUTPUT OPTIMUM CONDITION VALUES

END

# FIG.7

EP 4 485 298 A1

| EXPERI-MENT NUMBER | EXPERIMENT CONDITIONS | | | | | | | | | OPTIMUM CONDITIONS | | | | | EUCLIDEAN DISTANCE BETWEEN Nth TIME AND N-1th TIME | DIFFERENCE IN REACTION RATE UNDER OPTIMUM CONDITIONS (PREDICTED VALUE vs. ACTUAL REACTION VALUE) |
|---|---|---|---|---|---|---|---|---|---|---|---|---|---|---|---|---|
| | SYN-THESIS SCALE | AMIDITE | | ACTIVATING AGENT | | TEMPER-ATURE OF SUPPLIED LIQUID | RECYCLE TIME | RECYCLE FLOW RATE | REACTION RATE (ACTUAL REACTION VALUE) | AMOUNT OF CHARGED ACTI-VATING AGENT | TEMPER-ATURE OF SUPPLIED LIQUID | RECYCLE TIME | RECYCLE FLOW RATE | REACTION RATE (PRE-DICTED VALUE) | | |
| | | TYPE | CHARG-ED AMOUNT | TYPE | CHARG-ED AMOUNT | | | | | | | | | | | |
| | $\mu$ mol | | EQUIV-ALENT | | % | °C | min | cm/hr | % | % | °C | min | cm/hr | % | – | % |
| FIRST TIME | 480 | DNA | 1.4 | DCI | 50.0 | 22.5 | 10 | 212 | 74.3 | – | – | – | – | – | – | – |
| SECOND TIME | 480 | DNA | 1.4 | DCI | 50.0 | 15 | 5 | 106 | 67.0 | – | – | – | – | – | – | – |
| THIRD TIME | 480 | DNA | 1.4 | DCI | 50.0 | 30 | 15 | 424 | 80.6 | – | – | – | – | – | – | – |
| FOURTH TIME | 480 | DNA | 1.4 | DCI | 67.0 | 15 | 10 | 424 | 72.5 | – | – | – | – | – | – | – |
| FIFTH TIME | 480 | DNA | 1.4 | DCI | 67.0 | 22.5 | 15 | 106 | 79.3 | – | – | – | – | – | – | – |
| SIXTH TIME | 480 | DNA | 1.4 | DCI | 67.0 | 30 | 5 | 212 | 80.6 | – | – | – | – | – | – | – |
| SEV-ENTH TIME | 480 | DNA | 1.4 | DCI | 80.0 | 15 | 15 | 212 | 78.0 | – | – | – | – | – | – | – |
| EIGHTH TIME | 480 | DNA | 1.4 | DCI | 80.0 | 22.5 | 5 | 424 | 83.2 | – | – | – | – | – | – | – |
| NINTH TIME | 480 | DNA | 1.4 | DCI | 80.0 | 30 | 10 | 106 | 80.1 | 80 | 30 | 5 | 424 | 82 | – | – |
| TENTH TIME | 480 | DNA | 1.4 | DCI | 80.0 | 30 | 5 | 424 | 82.5 | 80 | 30 | 5 | 424 | 82 | 0 | – |
| ELEV-ENTH TIME | 480 | DNA | 1.4 | DCI | 80.0 | 30 | 5 | 424 | 82.3 | – | – | – | – | – | – | 0.4 |

| | INTERNATIONAL SEARCH REPORT | International application No. |
|---|---|---|
| | | **PCT/JP2023/006148** |

**A.    CLASSIFICATION OF SUBJECT MATTER**

*G06N 99/00*(2019.01)i
FI:    G06N99/00 180

According to International Patent Classification (IPC) or to both national classification and IPC

**B.    FIELDS SEARCHED**

Minimum documentation searched (classification system followed by classification symbols)

G06N99/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| Y | JP 2021-43959 A (TOKYO INST TECH) 18 March 2021 (2021-03-18) paragraphs [0009]-[0065] | 1-5, 7-9 |
| A | | 6 |
| Y | JP 2019-185591 A (HITACHI LTD) 24 October 2019 (2019-10-24) paragraph [0003] | 1-5, 7-9 |
| A | JP 2010-78084 A (TOYOTA MOTOR CORP) 08 April 2010 (2010-04-08) | 1-9 |

☐ Further documents are listed in the continuation of Box C.       ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 April 2023** | **16 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

| International application No. |
| --- |
| **PCT/JP2023/006148** |

| Patent document cited in search report | | | Publication date (day/month/year) | Patent family member(s) | Publication date (day/month/year) |
| --- | --- | --- | --- | --- | --- |
| JP | 2021-43959 | A | 18 March 2021 | US 2022/0309198 A1 paragraphs [0021]-[0080] WO 2021/044913 A1 | |
| JP | 2019-185591 | A | 24 October 2019 | (Family: none) | |
| JP | 2010-78084 | A | 08 April 2010 | (Family: none) | |

Form PCT/ISA/210 (patent family annex) (January 2015)

**EP 4 485 298 A1**

**Patent documents cited in the description**

- JP 2019215750 A
- JP 2022025318 A